(19) Europäisches Patentamt / European Patent Office / Office européen des brevets

(11) **EP 4 014 874 B1**

(12) **EUROPEAN PATENT SPECIFICATION**

(45) Date of publication and mention of the grant of the patent:
**13.12.2023 Bulletin 2023/50**

(21) Application number: **20859940.7**

(22) Date of filing: **21.07.2020**

(51) International Patent Classification (IPC):
***A61B 6/00*** *(2006.01)*

(52) Cooperative Patent Classification (CPC):
**A61B 6/482; A61B 6/4035; A61B 6/4042; A61B 6/405; A61B 6/42; A61B 6/4208; A61B 6/4233; A61B 6/4241; A61B 6/463; A61B 6/481; A61B 6/505; A61B 6/5205; A61B 6/5217; A61B 6/5235; A61B 6/5241;** (Cont.)

(86) International application number:
**PCT/JP2020/028194**

(87) International publication number:
**WO 2021/044754 (11.03.2021 Gazette 2021/10)**

(54) **IMAGE PROCESSING DEVICE, RADIOGRAPHIC IMAGING SYSTEM, IMAGE PROCESSING METHOD, AND PROGRAM**

BILDVERARBEITUNGSVORRICHTUNG, RADIOGRAFISCHES BILDGEBUNGSSYSTEM, BILDVERARBEITUNGSVERFAHREN UND PROGRAMM

DISPOSITIF DE TRAITEMENT D'IMAGES, SYSTÈME D'IMAGERIE RADIOGRAPHIQUE, PROCÉDÉ DE TRAITEMENT D'IMAGE ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2019 JP 2019159726**

(43) Date of publication of application:
**22.06.2022 Bulletin 2022/25**

(73) Proprietor: **CANON KABUSHIKI KAISHA OHTA-KU Tokyo 146-8501 (JP)**

(72) Inventors:
• **IWASHITA, Atsushi Tokyo 146-8501 (JP)**
• **TORII, Sota Tokyo 146-8501 (JP)**

• **TERUI, Kosuke Tokyo 146-8501 (JP)**

(74) Representative: **WESER & Kollegen Patentanwälte PartmbB Radeckestraße 43 81245 München (DE)**

(56) References cited:
**WO-A1-2014/188864      WO-A1-2019/069522**
**JP-A- H05 161 633      JP-A- 2008 229 122**
**JP-A- 2009 078 034      JP-A- 2019 110 955**
**US-A1- 2011 019 891      US-A1- 2013 129 180**
**US-A1- 2014 270 064      US-A1- 2019 159 741**

(52) Cooperative Patent Classification (CPC): (Cont.)
**H04N 5/32;** H04N 25/771

**Description**

TECHNICAL FIELD

The present invention relates to an image processing apparatus, a radiation imaging system, an image processing method, and a program. Specifically, the present invention relates to a radiation imaging apparatus and a radiation imaging system to be used in medical diagnosis for the capturing of still images, such as general radiography, and for the capturing of moving images, such as fluoroscopy.

BACKGROUND ART

In recent years, radiation imaging apparatuses in which flat panel detectors (hereinafter "FPDs") are used are being widely used as image-capturing apparatuses for use in radiation-based medical image diagnosis and non-destructive examination. Energy subtraction is an image-capturing method in which an FPD is used. In energy subtraction, thickness images for a plurality of materials, such as a bone image and a soft-tissue image for example, can be derived from a plurality of images with different radiation energies that are obtained by emitting radiation at different tube voltages, etc.

PTL 1 discloses a technique in which the image quality of a bone part image is improved by smoothing a soft-tissue image and subtracting the smoothed image from an accumulation image.
PTL 2 relates to estimating the thickness of an object that includes at least two materials, from a radiation image taken with radiations of at least two energy bands; and generating an image by comparing the estimated thickness to a thickness of a local region and extracting a region of interest. PTL 3 relates to modeling a body by generating thickness charts for different materials comprising the body on the basis of three images resulting from the passage through the body of X-rays with emission spectra that are distinct from one another.

CITATION LIST

PATENT LITERATURE

PTL 1: Japanese Patent Laid-Open No. H3-285475
PTL 2: US 2013/0129180 A1
PTL 3: US 2011/0019891 A1

SUMMARY OF THE DISCLOSURE

TECHNICAL PROBLEM

In interventional radiology (IVR) in which an FPD is used, a contrast agent is injected into blood vessels and medical devices such as a catheter and a guide wire are inserted into a blood vessel, and a treatment is performed while checking the positions and shapes of the contrast agent and the medical devices.

However, there is a problem that, if bone thickness and soft-tissue thickness are separated using energy subtraction, the results may include noise, which includes materials other than the separated materials.

In view of the above-described problem, the present invention provides an image processing technique that allows material decomposition images with reduced noise to be obtained by utilizing the continuity of thickness in the human body.

SOLUTION TO PROBLEM

An image processing apparatus according to one aspect of the present invention is defined in claim 1.

An image processing method according to another aspect of the present invention defined in claim 10. The other claims relate to further developments.

ADVANTAGEOUS EFFECTS OF INVENTION The invention is set out in the appended set of claims.

According to the present invention, material decomposition images with reduced noise can be obtained.

Other features and advantages of the present invention will be apparent from the following description taken in conjunction with the accompanying drawings. Note that the same reference numerals denote the same or like components throughout the accompanying drawings.

BRIEF DESCRIPTION OF DRAWINGS

The accompanying drawings, which are incorporated in and constitute a part of the specification, illustrate embodiments of the invention and, together with the description, serve to explain principles of the invention.

FIG. 1 is a diagram illustrating an example of a configuration of an X-ray image-capturing system according to a first embodiment.
FIG. 2 is an equivalent circuit diagram of a pixel of an X-ray imaging apparatus according to the first embodiment.
FIG. 3 is a timing chart of the X-ray imaging apparatus according to the first embodiment.
FIG. 4 is a timing chart of the X-ray imaging apparatus according to the first embodiment.
FIG. 5 is a diagram for describing correction processing according to the first embodiment.
FIG. 6 is a block diagram of signal processing according to the first embodiment.
FIG. 7 is a block diagram of image processing according to the first embodiment.
FIG. 8 is a diagram illustrating examples of an accumulation image and a bone image according to the first embodiment.
FIG. 9 is a diagram illustrating examples of a soft-tissue image and a thickness image according to the first embodiment.
FIG. 10 is a diagram illustrating examples of the accumulation image and the thickness image according to the first embodiment.
FIG. 11 includes portion 11A that is a diagram illustrating the relationship between X-ray spectra and energies, and portion 11B that is a diagram illustrating the relationship between linear attenuation coefficients and energies.
FIG. 12 is a block diagram of signal processing according to a second embodiment.
FIG. 13 is a block diagram of signal processing according to the second embodiment.
FIG. 14 is a block diagram of signal processing according to a third embodiment.
FIG. 15 is a block diagram of signal processing according to the third embodiment.

DESCRIPTION OF EMBODIMENTS

In the following, embodiments will be described in detail with reference to the attached drawings. Note that the following embodiments do not limit the invention according to the claims. While a plurality of features are described in the embodiments, it does not necessarily mean that all such features are necessary for the invention, and a plurality of features may also be combined as appropriate. Furthermore, the same reference numeral is appended in the attached drawings to configurations that are the same as or similar to one another, and overlapping description will be omitted.
Further note that the First Embodiment is a reference embodiment outside of the scope of the present invention.
Note that the term "radiation" in the present invention includes, in addition to alpha rays, beta rays, gamma rays, etc., which are beams formed by particles (including photons) that are emitted as a result of radioactive decay, beams having similar or higher energies, such as X-rays, particle beams, and cosmic rays. In the following embodiments, an apparatus in which X-rays are used as one example of radiation will be described. Accordingly, in the following, an X-ray imaging apparatus and an X-ray imaging system will be described as a radiation imaging apparatus and a radiation imaging system, respectively.

First Embodiment

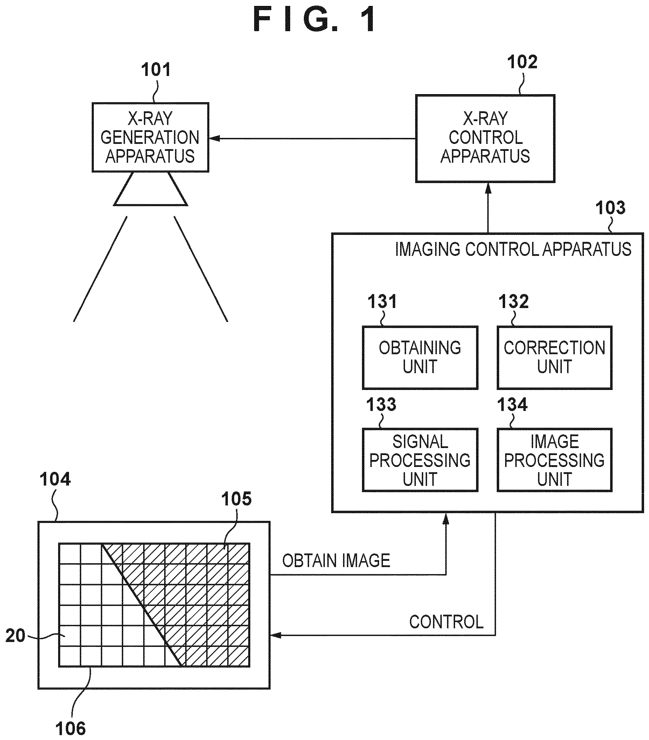

FIG. 1 is a block diagram illustrating an example of a configuration of an X-ray imaging system according to the first embodiment as one example of a radiation imaging system. The X-ray imaging system according to the first embodiment includes an X-ray generation apparatus 101, an X-ray control apparatus 102, an imaging control apparatus 103, and an X-ray imaging apparatus 104.
The X-ray generation apparatus 101 generates X-rays and irradiates objects with X-rays. The X-ray control apparatus 102 controls the generation of X-rays in the X-ray generation apparatus 101. The imaging control apparatus 103 includes at least one processor (CPU) and a memory, for example, and obtains X-ray images and performs image processing on the X-ray images by the processor executing one or more programs stored in the memory. Note that the processing performed by the imaging control apparatus 103, which includes the image processing, may be realized by dedicated hardware or by hardware and software working together. The X-ray imaging apparatus 104 includes a fluorescent material 105 that converts the X-rays into visible light, and a two-dimensional detector 106 that detects the visible light. The two-dimensional detector 106 is a sensor in which pixels 20 for detecting X-ray quanta are arranged in an array having X columns and Y rows, and outputs image information.
The imaging control apparatus 103 functions as an image processing apparatus that processes radiation images using the above-mentioned processor. Examples of functional configurations as an image processing apparatus are illustrated as an obtaining unit 131, a correction unit 132, a signal processing unit 133, and an image processing unit 134. The obtaining unit 131 obtains a plurality of radiation images with mutually different energies that are obtained by capturing images while irradiating the object with radiation. As the plurality of radiation images, the obtaining unit 131 obtains radiation images that are obtained by performing sampling and holding multiple times during exposure to one shot of radiation. The correction unit 132 corrects the plurality of radiation images obtained by the obtaining unit 131 and generates a plurality of images to be used in energy subtraction processing.

The signal processing unit 133 generates material characteristic images using the plurality of images generated by the correction unit 132. The material characteristic images are images obtained through the energy subtraction processing, such as material decomposition images for separately indicating materials such as bone and soft tissue, or material identification images indicating effective atomic numbers and surface densities thereof, for example. The signal processing unit 133 generates a first material decomposition image indicating a thickness of a first material and a second material decomposition image indicating a thickness of a second material based on a plurality of radiation images captured at mutually different radiation energies. The signal processing unit 133 generates a thickness image in which the thickness of the first material and the thickness of the second material are added together. Here, the first material includes at least one of calcium, hydroxyapatite, or bone, and the second material includes at least one of water, fat, or a soft material that does not contain calcium. The signal processing unit 133 will be described in detail later. The image processing unit 134 generates a display image using the material characteristic images obtained through the signal processing.

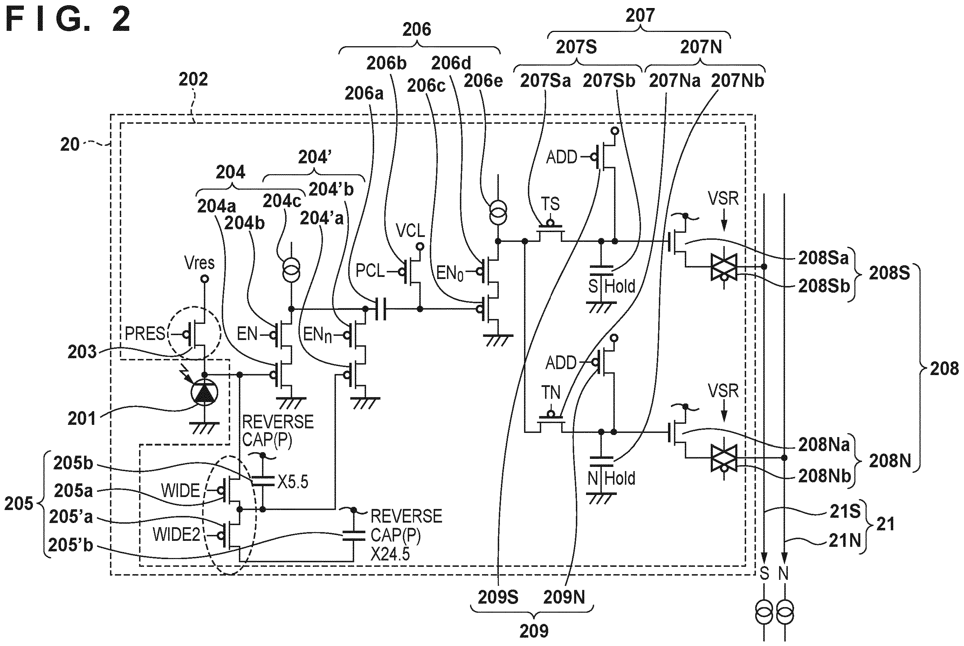

FIG. 2 is an equivalent circuit diagram of a pixel 20 according to the first embodiment. The pixel 20 includes a photoelectric conversion element 201 and an output circuit section 202. The photoelectric conversion element 201 may typically be a photodiode. The output circuit section 202 includes an amplifier circuit section 204, a clamp circuit section 206, a sample-and-hold circuit 207, and a selector circuit section 208.

The photoelectric conversion element 201 includes a charge accumulator, and the charge accumulator is connected to the gate of a MOS transistor 204a of the amplifier circuit section 204. The source of the MOS transistor 204a is connected to a current source 204c via a MOS transistor 204b. The MOS transistor 204a and the current source 204c constitute a source follower circuit. The MOS transistor 204b is an enabling switch that switches on and puts the source follower circuit in an operating state when an enable signal EN supplied to the gate of the MOS transistor 204b is set to an active level.

In the example illustrated in FIG. 2, the charge accumulator of the photoelectric conversion element 201 and the gate of the MOS transistor 204a form one same node, and this node functions as a charge-voltage converter that converts charge accumulated in the charge accumulator into a voltage. That is, a voltage $V$ ($=Q/C$) that is determined by the charge $Q$ accumulated in the charge accumulator and a capacitance value $C$ of the charge-voltage converter appears in the charge-voltage converter. The charge-voltage converter is connected to a reset potential Vres via a reset switch 203. When a reset signal PRES is set to an active level, the reset switch 203 switches on, and the potential of the charge-voltage converter is reset to the reset potential Vres.

The clamp circuit section 206 uses a clamp capacitor 206a and clamps noise that is output by the amplifier circuit section 204 in accordance with the reset potential of the charge-voltage converter. That is, the clamp circuit section 206 is a circuit for cancelling out this noise from a signal output from the source follower circuit in accordance with the charge generated by the photoelectric conversion element 201 through photoelectric conversion. This noise includes kTC noise generated when resetting is performed. The clamping is performed by setting a clamp signal PCL to an active level and switching a MOS transistor 206b on, and then setting the clamp signal PCL to a non-active level and switching the MOS transistor 206b off. The output side of the clamp capacitor 206a is connected to the gate of a MOS transistor 206c. The source of the MOS transistor 206c is connected to a current source 206e via a MOS transistor 206d. The MOS transistor 206c and the current source 206e constitute a source follower circuit. The MOS transistor 206d is an enabling switch that switches on and puts the source follower circuit in an operating state when an enable signal EN0 supplied to the gate of the MOS transistor 206d is set to an active level.

The signal that is output from the clamp circuit section 206 in accordance with the charge generated by the photoelectric conversion element 201 through photoelectric conversion is written as a light signal to a capacitor 207Sb via a switch 207Sa when a light-signal sampling signal TS is set to an active level. A signal that is output from the clamp circuit section 206 when the MOS transistor 206b is switched on immediately after the potential of the charge-voltage converter is reset is a clamp voltage. The noise signal is written to a capacitor 207Nb via a switch 207Na when a noise sampling signal TN is set to an active level. This noise signal includes an offset component of the clamp circuit section 206. The switch 207Sa and the capacitor 207Sb constitute a signal sample-and-hold circuit 207S, and the switch 207Na and the capacitor 207Nb constitute a noise sample-and-hold circuit 207N. The sample-and-hold circuit 207 includes the signal sample-and-hold circuit 207S and the noise sample-and-hold circuit 207N.

When a driving circuit section drives and sets a row selection signal to an active level, the signal (light signal) held in the capacitor 207Sb is output to a signal line 21S via a MOS transistor 208Sa and a row selection switch 208Sb. Furthermore, the signal (noise) held in the capacitor 207Nb is simultaneously output to a signal line 21N via a MOS transistor 208Na and a row selection switch 208Nb. The MOS transistor 208Sa, along with a constant current source (not illustrated) provided to the signal line 21S, constitutes a source follower circuit. Similarly, the MOS transistor 208Na, along with a constant current source (not illustrated) provided to the signal line 21N, constitute a source follower circuit. The MOS transistor 208Sa and the row selection switch 208Sb constitute a signal selector circuit section 208S, and the MOS transistor 208Na and the row selection switch 208Nb constitute a noise selector circuit section 208N. The selector circuit section 208 includes the signal selector circuit section 208S and the noise selector circuit section 208N.

The pixel 20 may include an adding switch 209S for adding light signals of a plurality of adjacent pixels 20. During an adding mode, an adding mode signal ADD is set to an active level, and the adding switch 209S is switched on. Thus, the capacitors 207Sb of adjacent pixels 20 are mutually connected by the adding switch 209S, and the light signals are averaged. Similarly, the pixel 20 may include an adding switch 209N for adding noises of a plurality of adjacent pixels 20. When the adding switch 209N switches on, the capacitors 207Nb of adjacent pixels 20 are mutually connected by the adding switch 209N, and the noises are averaged. An adding section 209 includes the adding switch 209S and the adding switch 209N.

Furthermore, the pixel 20 may include a sensitivity changing section 205 for changing sensitivity. For example, the pixel 20 may include a first sensitivity changing switch 205a and a second sensitivity changing switch 205'a, and circuit elements accompanying these switches. When a first change signal WIDE is set to an active level, the first sensitivity changing switch 205a switches on, and the capacitance value of a first additional capacitor 205b is added to the capacitance value of the charge-voltage converter. Thus, the sensitivity of the pixel 20 decreases. When a second change signal WIDE2 is set to an active level, the second sensitivity changing switch 205'a switches on, and the capacitance value of a second additional capacitor 205'b is added to the capacitance value of the charge-voltage converter. Thus, the sensitivity of the pixel 20 decreases to a further extent. By adding a function of decreasing the sensitivity of the pixel 20 in such manner, the pixel 20 can receive a larger light amount and the dynamic range thereof can be widened. If the first change signal WIDE is set to an active level, an enable signal ENn may be set to an active level to cause a MOS transistor 204'a to perform a source follower operation in place of the MOS transistor 204a.

The X-ray imaging apparatus 104 reads, from the two-dimensional detector 106, the outputs from pixel circuits as described above, converts the outputs into digital values using an AD converter (not illustrated), and then transfers an image to the imaging control apparatus 103.

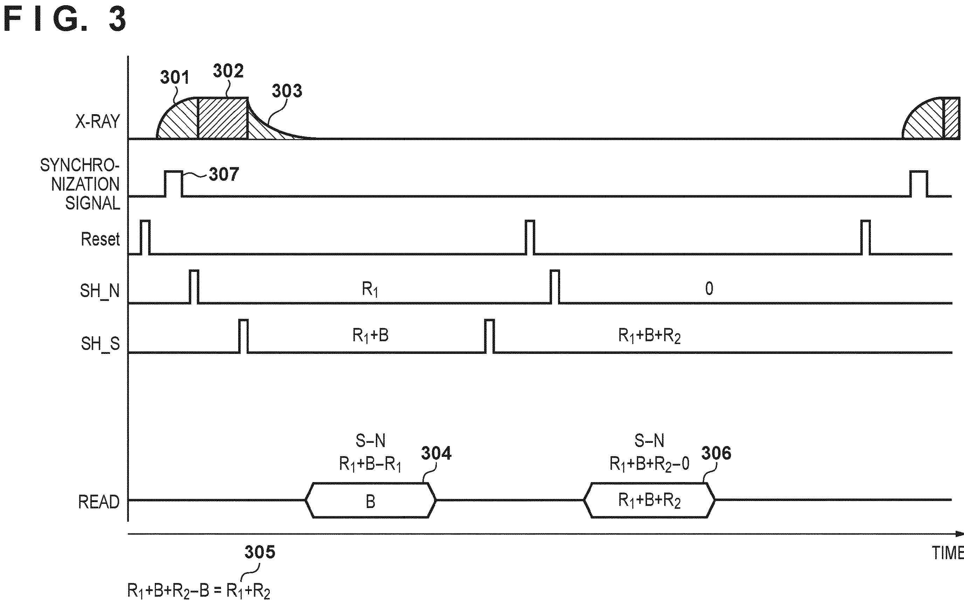

Next, the operation of the X-ray imaging system according to the first embodiment having the above-described configuration will be described. FIG. 3 illustrates driving timings of the X-ray imaging apparatus 104 for obtaining a plurality of X-ray images with mutually different energies that are to be provided to the energy subtraction in the X-ray imaging system according to the first embodiment. With the horizontal axis indicating time, the waveforms in FIG. 3 indicate: X-ray exposure; a synchronization signal; the resetting of the photoelectric conversion element 201; the sample-and-hold circuit 207; and timings when images are read from the signal lines 21.

X-ray exposure is performed after the photoelectric conversion element 201 is reset based on the reset signal. The X-ray tube voltage is ideally a square wave. However, it takes a finite amount of time for the tube voltage to rise and fall. Particularly in a case in which a pulse X-ray beam is used and the exposure time is short, the tube voltage can no longer be regarded as a square wave, and exhibits a waveform as indicated by X-rays 301 to 303. Rise period X-rays 301, stable period X-rays 302, and fall period X-rays 303 have different X-ray energies. Thus, by obtaining X-ray images corresponding to radiation during periods separated by performing sampling and holding, a plurality of types of X-ray images with mutually different energies can be obtained.

The X-ray imaging apparatus 104 performs sampling using the noise sample-and-hold circuit 207N after exposure to the rise period X-rays 301, and further performs sampling using the signal sample-and-hold circuit 207S after exposure to the stable period X-rays 302. Subsequently, the X-ray imaging apparatus 104 reads the difference between the signal line 21N and the signal line 21S as an image. Here, a signal (Ri) of the rise period X-rays 301 is held in the noise sample-and-hold circuit 207N, and the sum (Ri+B) of the signal of the rise period X-rays 301 and a signal (B) of the stable period X-rays 302 is held in the signal sample-and-hold circuit 207S. Accordingly, an image 304 that corresponds to the signal of the stable period X-rays 302 is read.

Next, the X-ray imaging apparatus 104 performs sampling using the signal sample-and-hold circuit 207S again after the exposure to the fall period X-rays 303 and the reading of the image 304 are completed. Subsequently, the X-ray imaging apparatus 104 resets the photoelectric conversion element 201, performs sampling using the noise sample-and-hold circuit 207N again, and reads the difference between the signal line 21N and the signal line 21S as an image. Here, a signal of a state in which no X-ray exposure is performed is held in the noise sample-and-hold circuit 207N, and the sum ($R_1+B+R_2$) of the signal of the rise period X-rays 301, the stable period X-rays 302, and a signal ($R_2$) of the fall period X-rays 303 is held in the signal sample-and-hold circuit 207S. Accordingly, an image 306 that corresponds to the signal of the rise period X-rays 301, the signal of the stable period X-rays 302, and the signal of the fall period X-rays 303 is read. Subsequently, by calculating the difference between the image 306 and the image 304, an image 305 that corresponds to the sum of the rise period X-rays 301 and the fall period X-rays 303 is obtained. This calculation may be performed by the X-ray imaging apparatus 104 or by the imaging control apparatus 103.

The timings when the sample-and-hold circuit 207 and the photoelectric conversion element 201 are reset are determined using a synchronization signal 307 that indicates that the exposure to X-rays from the X-ray generation apparatus 101 has started. A configuration of measuring the tube current of the X-ray generation apparatus 101 and determining whether or not the current value exceeds a preset threshold may be adopted as the method for detecting the start of X-ray exposure, but the method for detecting the start of X-ray exposure is not limited to this. For example, a configuration may be adopted in which the start of X-ray exposure is detected by, after the resetting of the photoelectric conversion element 201 is completed, repeating reading from the pixel 20 and determining whether or not the pixel value exceeds a preset threshold.

Alternatively, a configuration may be adopted in which an X-ray detector that is different from the two-dimensional detector 106 is built into the X-ray imaging apparatus 104, and the start of X-ray exposure is detected by determining whether or not a measurement value of the X-ray detector exceeds a preset threshold, for example. In any case, the sampling using the signal sample-and-hold circuit 207S, the sampling using the noise sample-and-hold circuit 207N, and the resetting of the photoelectric conversion element 201 are performed after a predetermined amount of time elapses from when the synchronization signal 307 indicating the start of X-ray exposure is input.

In such a manner, the image 304 corresponding to the stable period of a pulse X-ray beam and the image 305 corresponding to the sum of the rise period and the fall period of the pulse X-ray beam are obtained. These two X-ray images are formed through exposure to X-rays having mutually different energies, and thus the energy subtraction processing can be performed by performing calculation between these X-ray images.

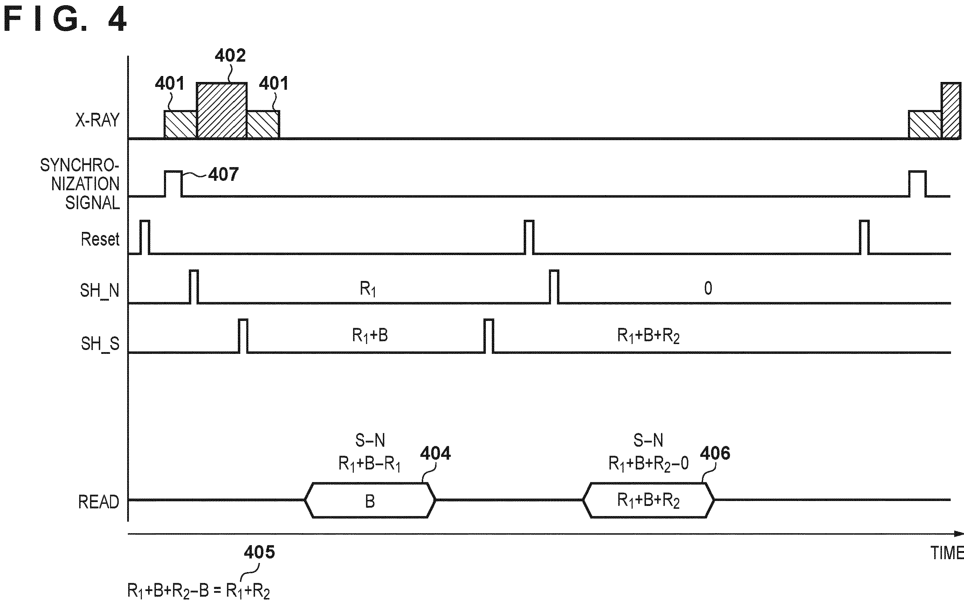

FIG. 4 illustrates driving timings of the X-ray imaging apparatus 104, differing from those in FIG. 3, for obtaining a plurality of X-ray images with mutually different energies that are to be provided to the energy subtraction in the X-ray imaging system according to the first embodiment. FIG. 4 differs from FIG. 3 in that the tube voltage of the X-ray generation apparatus 101 is actively switched.

First, the X-ray generation apparatus 101 performs exposure to low-energy X-rays 401 after the photoelectric conversion element 201 is reset. In this state, the X-ray imaging apparatus 104 performs sampling using the noise sample-and-hold circuit 207N. Subsequently, the X-ray generation apparatus 101 switches the tube voltage and performs exposure to high-energy X-rays 402. In this state, the X-ray imaging apparatus 104 performs sampling using the signal sample-and-hold circuit 207S. Subsequently, the X-ray generation apparatus 101 switches the tube voltage and performs exposure to low-energy X-rays 401. The X-ray imaging apparatus 104 reads the difference between the signal line 21N and the signal line 21S as an image. Here, a signal (Ri) of the low-energy X-rays 401 is held in the noise sample-and-hold circuit 207N, and the sum (Ri+B) of the signal of the low-energy X-rays 401 and a signal (B) of the high-energy X-rays 402 is held in the signal sample-and-hold circuit 207S. Accordingly, an image 404 that corresponds to the signal of the high-energy X-rays 402 is read.

Next, the X-ray imaging apparatus 104 performs sampling using the signal sample-and-hold circuit 207S again after the exposure to the low-energy X-rays 403 and the reading of the image 404 are completed. Subsequently, the X-ray imaging apparatus 104 resets the photoelectric conversion element 201, performs sampling using the noise sample-and-hold circuit 207N again, and reads the difference between the signal line 21N and the signal line 21S as an image. Here, a signal of a state in which no X-ray exposure is performed is held in the noise sample-and-hold circuit 207N, and the sum $(R_1+B+R_2)$ of the signal of the low-energy X-rays 401, the high-energy X-rays 402, and a signal $(R_2)$ of the low-energy X-rays 403 is held in the signal sample-and-hold circuit 207S. Accordingly, an image 406 that corresponds to the signal of the low-energy X-rays 401, the signal of the high-energy X-rays 402, and the signal of the low-energy X-rays 403 is read.

Subsequently, by calculating the difference between the image 406 and the image 404, an image 405 that corresponds to the sum of the low-energy X-rays 401 and the low-energy X-rays 403 is obtained. This calculation may be performed by the X-ray imaging apparatus 104 or by the imaging control apparatus 103. The synchronization signal (407) is similar to that in FIG. 3. By obtaining images while actively switching the tube voltage in such a manner, the energy difference between the low-energy and high-energy radiation images can be increased to a further extent compared to the method in FIG. 3.

Next, the energy subtraction processing by the imaging control apparatus 103 will be described. The energy subtraction processing in the first embodiment is divided into three stages, namely correction processing by the correction unit 132, signal processing by the signal processing unit 133, and image processing by the image processing unit 134. Each processing will be described below.

Description of Correction Processing

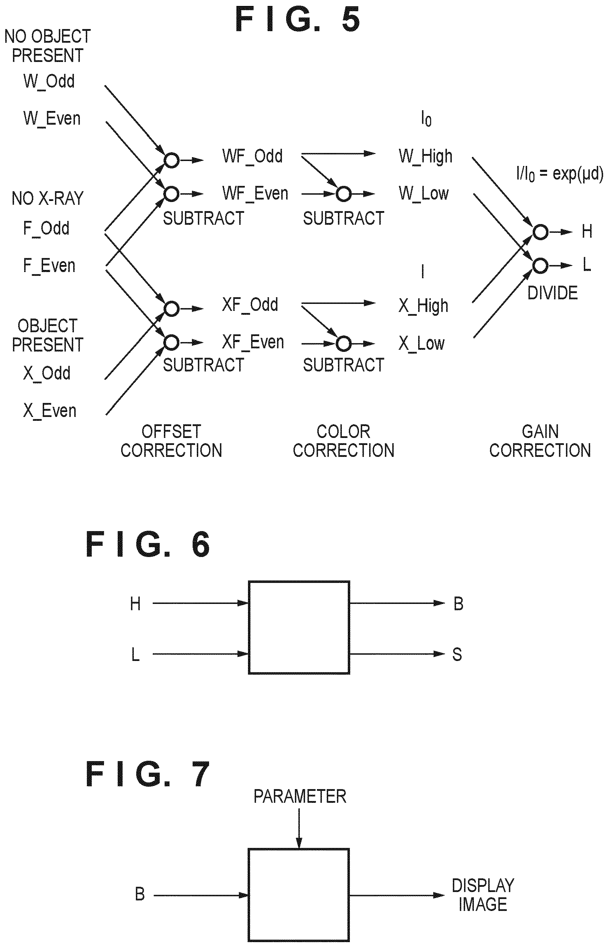

The correction processing is processing in which the plurality of radiation images obtained from the X-ray imaging apparatus 104 are processed to generate a plurality of images to be used in the later-described signal processing in the energy subtraction processing. FIG. 5 illustrates the correction processing for the energy subtraction processing according to the first embodiment. First, the obtaining unit 131 causes the X-ray imaging apparatus 104 to capture images in a state in which no X-ray exposure is performed, and obtains images according to the driving illustrated in FIG. 3 or FIG. 4. Two images are read as a result of the driving. In the following, the first image (image 304 or image 404) is referred to as F_ODD, and the second image (image 306 or image 406) is referred to as F_EVEN. F_ODD and F_EVEN are each an image corresponding to fixed pattern noise (FPN) of the X-ray imaging apparatus 104.

Next, the obtaining unit 131 exposes the X-ray imaging apparatus 104 to X-rays in a state in which no object is present and causes the X-ray imaging apparatus 104 to capture images to obtain gain correction images that are output from the X-ray imaging apparatus 104 according to the driving illustrated FIG. 3 or FIG. 4. As in the above-described case, two images are read as a result of this driving. In the following, the first gain-correction image (image 304 or image 404) is referred to as W_ODD, and the second gain-correction image (image 306 or image 406) is referred to as W_EVEN. W_ODD and W_EVEN are each an image corresponding to the sum of the FPN of the X-ray imaging apparatus 104 and an X-ray-based signal. The correction unit 132 obtains images WF_ODD and WF_EVEN from which the FPN of the X-ray imaging apparatus 104 has been removed by subtracting F_ODD from W_ODD and subtracting F_EVEN from W_EVEN. This is referred to as offset correction.

WF_ODD is an image that corresponds to the stable period X-rays 302, and WF_EVEN is an image that corresponds to the sum of the rise period X-rays 301, the stable period X-rays 302, and the fall period X-rays 303. Accordingly, the correction unit 132 obtains an image that corresponds to the sum of the rise period X-rays 301 and the fall period X-rays 303 by subtracting WF_ODD from WF_EVEN. Processing in which images that correspond to X-rays of specific periods separated by performing sampling and holding are obtained by performing subtraction using a plurality of images in such a manner is referred to as color correction. The energies of the rise period X-rays 301 and the fall period X-rays 303 are lower than the energy of the stable period X-rays 302. Accordingly, as a result of the color correction, a low-energy image W_LOW corresponding to a case in which no object is present can be obtained by subtracting WF_ODD from WF_EVEN. Furthermore, a high-energy image W_High corresponding to a case in which no object is present obtained from WF_ODD.

Next, the obtaining unit 131 exposes the X-ray imaging apparatus 104 to X-rays in a state in which an object is present and causes the X-ray imaging apparatus 104 to capture images to obtain images that are output from the X-ray imaging apparatus 104 according to the driving illustrated FIG. 3 or FIG. 4. Here, two images are read. In the following, the first image (image 304 or image 404) is referred to as X_ODD, and the second image (image 306 or image 406) is referred to as X_EVEN. By performing offset correction and color correction similar to those in the case in which no object is present, the correction unit 132 obtains a low-energy image X_Low corresponding to a case in which the object is present and a high-energy image X_High corresponding to a case in which the object is present.

Here, [Math. 1] below holds true, where d is the thickness of the object, $\mu$ is a linear attenuation coefficient of the object, $I_0$ is the output from the pixels 20 in a case in which no object is present, and I is the output from the pixels 20 in a case in which the object is present.

[Math. 1]

$$I = I_0 \exp(\mu d)$$

[Math. 2] below can be obtained by transforming [Math. 1]. The right side of [Math. 2] indicates the attenuation rate of the object. The attenuation rate of the object is a real number between 0 and 1.

[Math. 2]

$$I / I_0 = \exp(\mu d)$$

Accordingly, the correction unit 132 obtains a low-energy attenuation rate image L (hereinafter also referred to as "low-energy image L") by dividing the low-energy image X_Low corresponding to a case in which the object is present by the low-energy image W_Low corresponding to a case in which no object is not present. Similarly, the correction unit 132 obtains a high-energy attenuation rate image H (hereinafter also referred to as "high-energy image H") by dividing the high-energy image X_High corresponding to a case in which the object is present by the high-energy image W_High corresponding to a case in which no object is not present. Processing in which attenuation rate images are obtained by dividing images obtained based on radiation images obtained in a state in which the object is present by images obtained based on radiation images obtained in a state in which no object is present in such a manner is referred to as gain correction. This concludes the description of the correction processing by the correction unit 132 according to the first embodiment.

Description of Signal Processing

FIG. 6 illustrates a block diagram of the signal processing in the energy subtraction processing according to the first embodiment. The signal processing unit 133 generates material characteristic images using the plurality of images obtained from the correction unit 132. In the following, the generation of material decomposition images including a bone thickness image B and a soft-tissue thickness image S will be described. The signal processing unit 133, by performing the following processing, derives a bone thickness image B and a soft-tissue thickness image S from the low-energy attenuation rate image L and the high-energy attenuation rate image H obtained through the correction illustrated in FIG. 5.

First, [Math. 3] below holds true, where E is the X-ray photon energy, N(E) is the number of photons at the energy E, B is the thickness in the bone thickness image, S is the thickness in the soft-tissue thickness image, $\mu_B(E)$ is a linear attenuation coefficient of bone at the energy E, $\eta_S(E)$ is a linear attenuation coefficient of soft tissue at the energy E, and $I/I_0$ is the attenuation rate.

[Math. 3]

$$I / I_0 = \frac{\int_0^\infty N(E)\exp\{-\mu_B(E)B - \mu_S(E)S\}EdE}{\int_0^\infty N(E)EdE}$$

The number of photons N(E) at the energy E indicates an X-ray spectrum. The X-ray spectrum can be obtained through simulation or actual measurement. Furthermore, the linear attenuation coefficient $\mu_B(E)$ of bone at the energy E and the linear attenuation coefficient $\eta_S(E)$ of soft tissue at the energy E can be obtained from a database such as that provided by the National Institute of Standards and Technology (NIST). Accordingly, based on [Math. 3], the thickness B in any bone thickness image, the thickness S in any soft-tissue thickness image, and the attenuation rate $I/I_0$ for any X-ray spectrum N(E) can be calculated.

Here, the equations in [Math. 4] below hold true, where $N_L(E)$ is the low-energy X-ray spectrum, and $N_H(E)$ is the high-energy X-ray spectrum. Note that L is a pixel value in the low-energy attenuation rate image, and H is a pixel value in the high-energy attenuation rate image.

[Math. 4]

$$L = \frac{\int_0^\infty N_L(E)\exp\{-\mu_B(E)B - \mu_S(E)S\}EdE}{\int_0^\infty N_L(E)EdE}$$

$$H = \frac{\int_0^\infty N_H(E)\exp\{-\mu_B(E)B - \mu_S(E)S\}EdE}{\int_0^\infty N_H(E)EdE}$$

By solving the non-linear system of equations in [Math. 4], the thickness B in the bone thickness image and the thickness S in the soft-tissue thickness image can be derived. Here, a case will be described in which the Newton-Raphson method is used as a representative method for solving non-linear systems of equations. First, when m is the number of iterations of the Newton-Raphson method, $B^m$ is the bone thickness after the mth iteration, and $S^m$ is the soft-tissue thickness after the mth iteration, the high-energy attenuation rate $H^m$ after the mth iteration and the low-energy attenuation rate $L^m$ after the mth iteration can be expressed as in [Math. 5] below.

[Math. 5]

$$L^m = \frac{\int_0^{\infty} N_L(E)\exp\{-\mu_B(E)B^m - \mu_S(E)S^m\}EdE}{\int_0^{\infty} N_L(E)EdE}$$

$$H^m = \frac{\int_0^{\infty} N_H(E)\exp\{-\mu_B(E)B^m - \mu_S(E)S^m\}EdE}{\int_0^{\infty} N_H(E)EdE}$$

Furthermore, the rate of change in attenuation rate when there is a minute change in thickness is expressed using [Math. 6] below.

[Math. 6]

$$\frac{\partial H^m}{\partial B^m} = \frac{\int_0^{\infty} -\mu_B(E)N_H(E)\exp\{-\mu_B(E)B^m - \mu_S(E)S^m\}EdE}{\int_0^{\infty} N_H(E)EdE}$$

$$\frac{\partial L^m}{\partial B^m} = \frac{\int_0^{\infty} -\mu_B(E)N_L(E)\exp\{-\mu_B(E)B^m - \mu_S(E)S^m\}EdE}{\int_0^{\infty} N_L(E)EdE}$$

$$\frac{\partial H^m}{\partial S^m} = \frac{\int_0^{\infty} -\mu_S(E)N_H(E)\exp\{-\mu_B(E)B^m - \mu_S(E)S^m\}EdE}{\int_0^{\infty} N_H(E)EdE}$$

$$\frac{\partial L^m}{\partial S^m} = \frac{\int_0^{\infty} -\mu_S(E)N_L(E)\exp\{-\mu_B(E)B^m - \mu_S(E)S^m\}EdE}{\int_0^{\infty} N_L(E)EdE}$$

Here, the bone thickness $B^{m+1}$ and the soft-tissue thickness $S^{m+1}$ after the (m+1)th iteration are expressed using the high-energy attenuation rate H and the low-energy attenuation rate L as shown in [Math. 7] below.

[Math. 7]

$$\begin{bmatrix} B^{m+1} \\ S^{m+1} \end{bmatrix} = \begin{bmatrix} B^m \\ S^m \end{bmatrix} + \begin{bmatrix} \dfrac{\partial H^m}{\partial B^m} & \dfrac{\partial H^m}{\partial S^m} \\ \dfrac{\partial L^m}{\partial B^m} & \dfrac{\partial L^m}{\partial S^m} \end{bmatrix}^{-1} \begin{bmatrix} H - H^m \\ L - L^m \end{bmatrix}$$

According to the Cramer's rule, the inverse matrix of the 2×2 matrix can be expressed using [Math. 8] below, where det is the determinant.

[Math. 8]

$$\det = \frac{\partial H^m}{\partial B^m} \frac{\partial L^m}{\partial S^m} - \frac{\partial H^m}{\partial S^m} \frac{\partial L^m}{\partial B^m}$$

$$\begin{bmatrix} \dfrac{\partial H^m}{\partial B^m} & \dfrac{\partial H^m}{\partial S^m} \\ \dfrac{\partial L^m}{\partial B^m} & \dfrac{\partial L^m}{\partial S^m} \end{bmatrix}^{-1} = \frac{1}{\det} \begin{bmatrix} \dfrac{\partial L^m}{\partial S^m} & -\dfrac{\partial H^m}{\partial S^m} \\ -\dfrac{\partial L^m}{\partial B^m} & \dfrac{\partial H^m}{\partial B^m} \end{bmatrix}$$

Accordingly, [Math. 9] below can be derived by substituting [Math. 8] into [Math. 7].

[Math. 9]

$$B^{m+1} = B^m + \frac{1}{\det} \frac{\partial L^m}{\partial S^m} \left( H - H^m \right) - \frac{1}{\det} \frac{\partial H^m}{\partial S^m} \left( L - L^m \right)$$

$$S^{m+1} = S^m - \frac{1}{\det} \frac{\partial L^m}{\partial B^m} \left( H - H^m \right) + \frac{1}{\det} \frac{\partial H^m}{\partial B^m} \left( L - L^m \right)$$

By repeating such a calculation, the difference between the high-energy attenuation rate $H^m$ after the mth iteration and the actually-measured high-energy attenuation rate H infinitely approaches 0. The same applies also to the low-energy attenuation rate L. Thus, the bone thickness $B^m$ after the mth iteration converges to a bone thickness B, and the soft-tissue thickness $S^m$ after the mth iteration converges to a soft-tissue thickness S. The non-linear system of equations shown in [Math. 4] can be solved in such a manner. Accordingly, by calculating [Math. 4] for all pixels, a bone thickness image B and a soft-tissue thickness image S can be obtained from the low-energy attenuation rate image L and the high-energy attenuation rate image H.

Note that, while the bone thickness image B and the soft-tissue thickness image S are calculated in the first embodiment, the present invention is not limited to such an embodiment. For example, the thickness W of water and the thickness I of a contrast agent may be calculated. That is, decomposition may be performed into the thicknesses of any two kinds of materials. Furthermore, an image of effective atomic numbers Z and an image of surface densities D may be derived from the low-energy attenuation rate image L and the high-energy attenuation rate image H obtained through the corrections illustrated in FIG. 5. An effective atomic number Z is an atomic number equivalent of a mixture. Also, a surface density D is the product of object density [g/cm$^3$] and object thickness [cm].

Furthermore, the non-linear system of equations is solved using the Newton-Raphson method in the first embodiment. However, the present invention is not limited to such an embodiment. For example, iterative solution methods such as the least-squares method and the bisection method may be used. Furthermore, while the non-linear system of equations is solved using an iterative solution method in the first embodiment, the present invention is not limited to such an embodiment. A configuration may be adopted in which bone thicknesses B and soft-tissue thicknesses S for various combinations of the high-energy attenuation rate H and the low-energy attenuation rate L are derived in advance to create a table, and the bone thickness B and the soft-tissue thickness S are derived at high speed by referring to this table.

Description of Image Processing

FIG. 7 illustrates a block diagram of the image processing in the energy subtraction processing according to the first embodiment. The image processing unit 134 according to the first embodiment generates a display image by performing post-processing, etc., on the bone thickness image B obtained through the signal processing illustrated in FIG. 6. The image processing unit 134 may use logarithmic conversion, dynamic range compression, etc., as post-processing.

Furthermore, an accumulation image A, which is an image that is the sum of high energy and low energy, may be used as the display image, for example. The accumulation image A is an image that is compatible with images without energy resolution captured using existing radiation imaging systems. The image processing unit 134 may generate the accumulation image A by multiplying the high-energy attenuation rate image H and the low-energy attenuation rate image L by coefficients and adding the results. Alternatively, the image processing unit 134 may generate the accumulation image A by dividing the image X_EVEN illustrated in FIG. 5, which corresponds to the sum of the rise period X-rays 301, the stable period X-rays 302, and the fall period X-rays 303 of the X-rays in a case in which the object is present, by the image W_EVEN illustrated in FIG. 5, which corresponds to the sum of the rise period X-rays 301, the stable period X-rays 302, and the fall period X-rays 303 of the X-rays in a case in which no object is present.

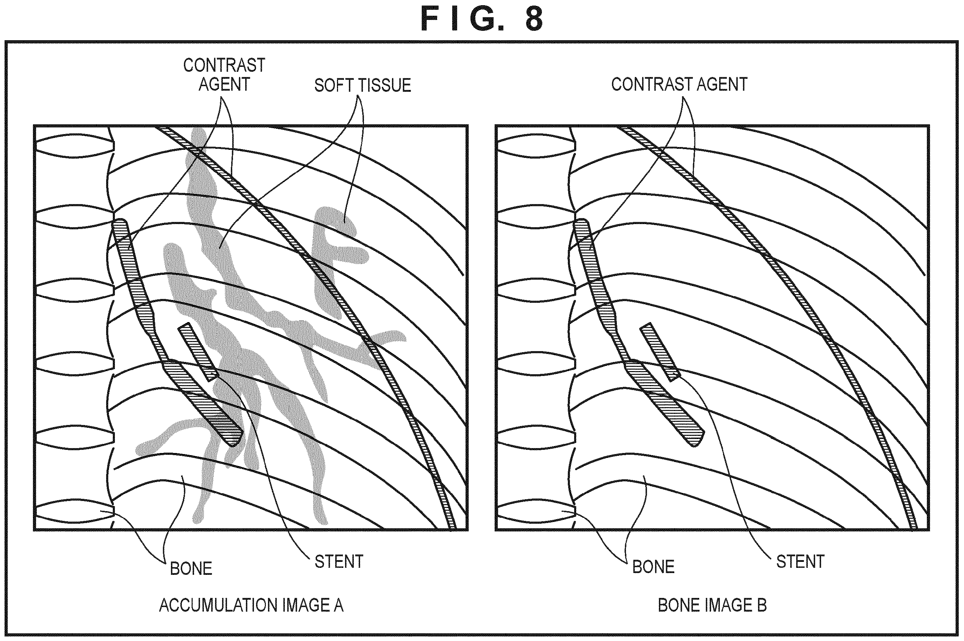

FIG. 8 is a diagram illustrating examples of the accumulation image A and the bone image B according to the first embodiment. A human body is normally formed from only soft tissue and bones. However, when IVR is performed using the radiation imaging system illustrated in FIG. 1, a contrast agent is injected into blood vessels. Furthermore, a catheter and a guide wire are inserted into a blood vessel to perform a procedure such as stenting or coiling. IVR is performed while checking the positions and shapes of the contrast agent and the medical devices. Accordingly, visibility may improve by separating only the contrast agent or the medical devices, or by removing backgrounds such as soft tissue and bones.

As illustrated in FIG. 8, in an image that is compatible with ordinary radiation imaging systems, i.e., in the accumulation image A, soft tissue is displayed as well as the contrast agent, a stent, and bones. On the other hand, in the radiation imaging system according to the first embodiment, the influence of soft tissue can be reduced by displaying the bone image B.

Meanwhile, the main component of a contrast agent is iodine, and the main component of a medical device is a metal, such as stainless steel. Both such materials have atomic numbers higher than that of calcium, which is the main component of bone, and thus bones, contrast agents, and medical devices are displayed in the bone image B.

According to an investigation carried out by the present inventors, even in a case such as that in which separation into a water image W and a contrast agent image I was performed based on the high-energy image H and the low-energy image L, bones, contrast agents, and medical devices were displayed in the contrast agent image I. Furthermore, this is the same even if the filters and tube voltages used to generate low-energy and high-energy X-rays are changed. That is, contrast agents and medical devices could not be separated from bones.

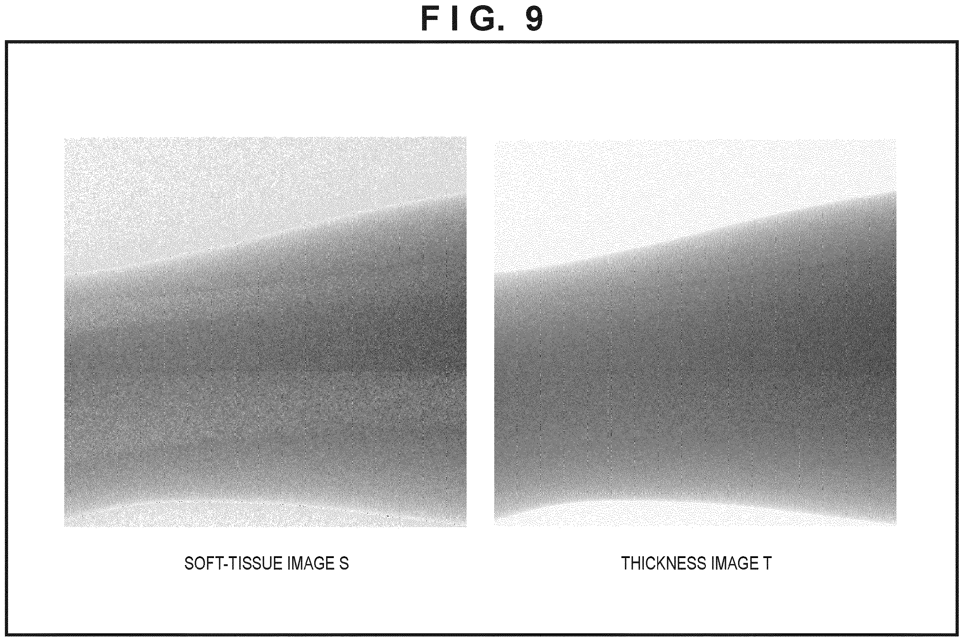

FIG. 9 is a diagram illustrating examples of the soft-tissue image and a thickness image according to the first embodiment. According to an observation carried out by the present inventors of soft-tissue images S of phantoms of the four limbs, it was found that a bone is visible as a decrease in soft tissue thickness. This is because the soft-tissue thickness decreases by an amount corresponding to the thickness of a bone. Furthermore, it was found through an observation of an image that is the sum of the bone image B and the soft-tissue image S, i.e., the thickness image T, that the bone contrast disappeared and was no longer visible. This is because the decrease in soft-tissue thickness in an area where a bone is present is offset by the thickness of the bone being added.

According to an investigation carried out by the present inventors, it was found that, while there are areas in the bones of the human body that do not contain calcium, such as the spongy bone and the bone marrow, the insides of such areas are filled with organic matter (i.e., not filled with gas). That is, it can be said that a human body has continuous thickness when projected from one direction. Thus, the contrast of a bone can be eliminated in the thickness image T even in the case of the human body. Here, it should be noted that the above-described continuity of thickness does not hold true for areas that may contain gases, such as the lungs and the digestive organs. Furthermore, it should also be noted that the inside of the spongy bone and the bone marrow is hollow (i.e., is filled with gases) in a dry human-bone phantom, and that there are creatures having hollow bones, such as birds.

In the present embodiment, the signal processing unit 133 generates a thickness image in which a thickness of a first material and a thickness of a second material are added together. Thus, a material decomposition image with reduced noise can be generated.

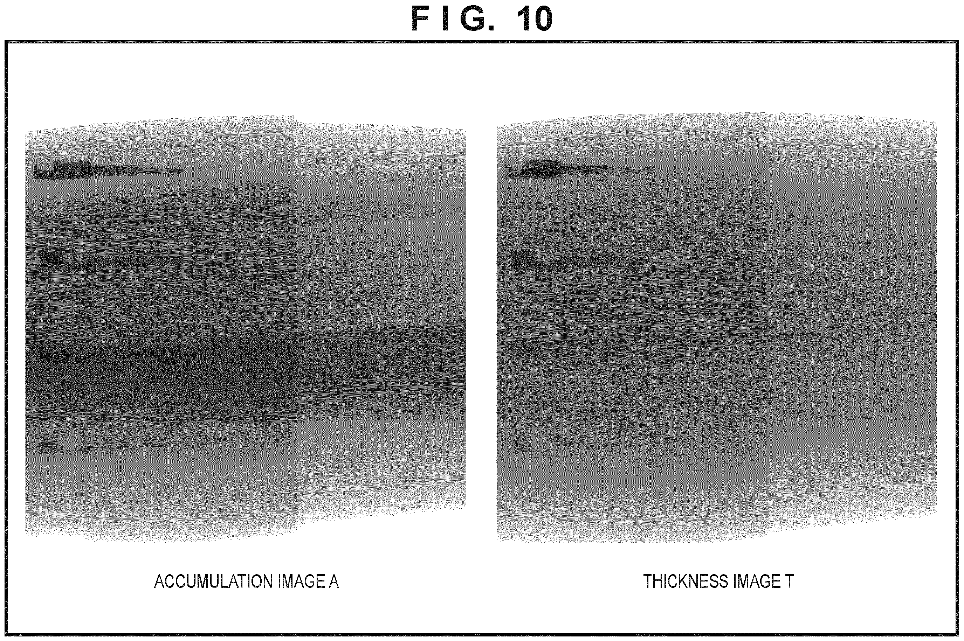

FIG. 10 is a diagram illustrating examples of the accumulation image A and the thickness image T according to the first embodiment. When a contrast agent is injected into the four limbs, both the bones and the contrast agent are visible in the accumulation image A. However, in the thickness image T, the contrast of bones disappears, and it is possible to see only soft tissue and the contrast agent. Accordingly, it can be expected that visibility can be improved in a situation such as where bones would be in the way in viewing the contrast agent and medical devices. However, depending on the tube voltages of the low-energy and high-energy X-rays illustrated in FIG. 4, there are cases in which, in the thickness image T, the contrast-agent contrast becomes weak at the same time as the bone contrast disappears, and thus the contrast agent becomes difficult to see.

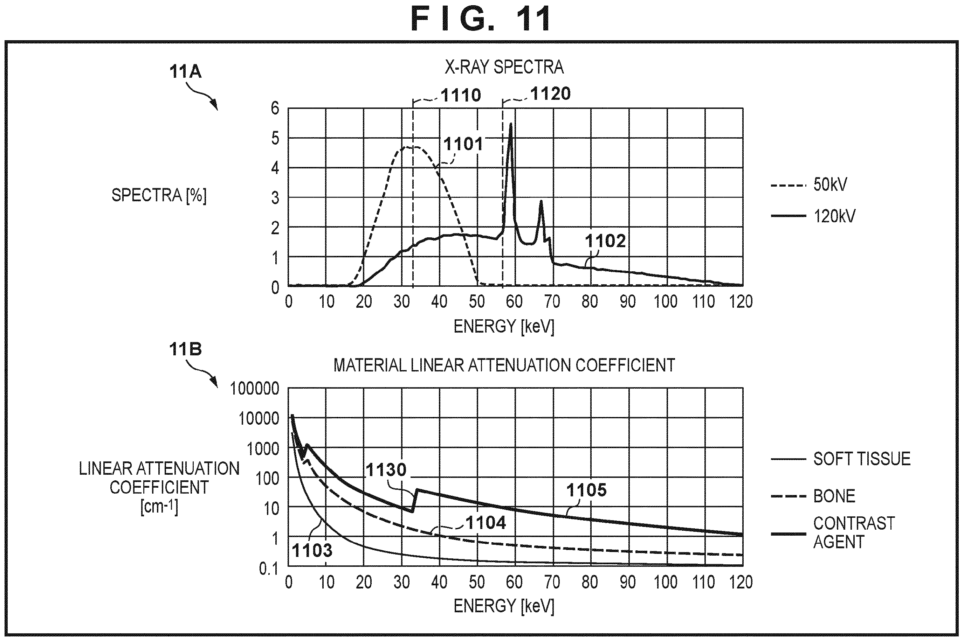

FIG. 11 includes portion 11A that is a diagram illustrating the relationship between X-ray spectra and energies, and portion 11B that is a diagram illustrating the relationship between linear attenuation coefficients and energies. In portion 11A of FIG. 11, a waveform 1101 indicates an X-ray spectrum at a 50-kV tube voltage, and a waveform 1102 indicates an X-ray spectrum at a 120-kV tube voltage. A broken line 1110 indicates the average energy (33 keV) of the X-ray spectrum at the 50-kV tube voltage, and a broken line 1120 indicates the average energy (57 keV) of the X-ray spectrum at the 120-kV tube voltage

Furthermore, as illustrated in portion 11B of FIG. 11, the linear attenuation coefficient varies depending on material (for example, soft tissue, bone, contrast agent, etc.) and energy. In portion 11B of FIG. 11, a waveform 1103 indicates the linear attenuation coefficient of soft tissue, a waveform 1104 indicates the linear attenuation coefficient of bone, and a waveform 1105 indicates the linear attenuation coefficient of a contrast agent.

In the present embodiment, the obtaining unit 131 obtains a plurality of radiation images by capturing images at a first energy (low energy) and at a second energy (high energy) that is higher than the first energy. Here, the average energy in the spectrum of radiation for obtaining radiation images based on the first energy is lower than the iodine K-edge 1130 (portion 11B of FIG. 11).

Generally, the greater the difference between the tube voltages of the low-energy and high-energy X-rays, the greater the difference between the linear attenuation coefficients of materials. Accordingly, the SN ratio of the images obtained through the signal processing illustrated in FIG. 6 is improved. On the other hand, the exposure dose necessary for achieving the same SN ratio tends to increase if the tube voltage of the low-energy X-rays is set too low. Thus, in the signal processing, the tube voltage of the low-energy X-rays and the tube voltage of the high-energy X-rays may be respectively set to 70 kV and 120 kV, etc., for example.

Here, as illustrated in portion 11B of FIG. 11 for example, the K-edge 1130 of iodine, which is the main component of contrast agents, is around 30 keV The contrast-agent contrast in the thickness image T can be emphasized by selecting a radiation quality such that this K-edge 1130 can be used. That is, the contrast-agent contrast in the thickness image T can be emphasized by selecting the radiation quality so that the average energy in the spectrum of the radiation for obtaining radiation images based on low energy is lower than the iodine K-edge.

For example, the signal processing unit 133 may use, in the signal processing, a radiation quality such as that obtained by setting the tube voltage of the low-energy X-rays to 40-50 kV and by not passing the X-rays through any additional filter. With such a radiation quality, the average energy of the low-energy X-rays falls below the iodine K-edge, and the contrast-agent contrast is readily emphasized. However, the low-energy X-rays hardly pass through thick objects. Accordingly, the first embodiment of the present invention is suitable for use with parts, such as the four limbs for example, which are relatively thin and in which the continuity of thickness illustrated in FIG. 9 favorably holds true.

In the image processing according to the present embodiment, the accumulation image A, the bone image B, or the thickness image T is displayed as the display image. However, the display image is not limited to such examples, and the image processing unit 134 may display the high-energy image H or the soft-tissue image S as the display image. Furthermore, the images obtained in the timing chart illustrated in FIG. 4, the images obtained in the correction processing illustrated in FIG. 5, and the images obtained through the signal processing illustrated in FIG. 6 may also be used. Furthermore, while logarithmic conversion and dynamic range compression have been mentioned as the post-processing to be applied to such images, there is no limitation to such an embodiment. For example, the image processing unit 134 may perform image processing such as the application of a temporal-direction filter such as a recursive filter or a spatial-direction filter such as a Gaussian filter. That is, it can be said that the image processing according to the present embodiment is processing in which images that have been captured, corrected, or signal-processed are subjected to calculation, as appropriate.

According to the present embodiment, material decomposition images with reduced noise can be obtained.

Second Embodiment Further note that the Second Embodiment is a reference embodiment outside of the scope of the present invention.

In the second embodiment, a configuration for utilizing the continuity of thickness illustrated in FIG. 9 and thereby reducing noise in the images (material decomposition images) obtained through the signal processing illustrated in FIG. 6 will be described.

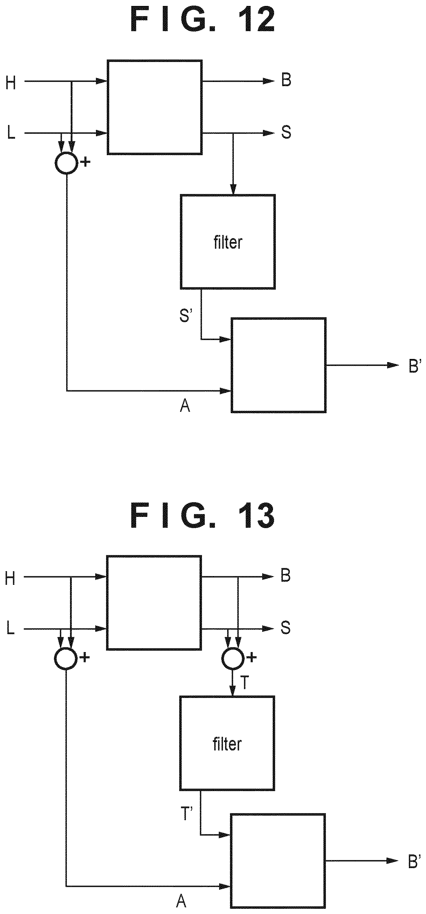

FIG. 12 illustrates a block diagram of signal processing according to the second embodiment. In the second embodiment, in a manner similar to that in the signal processing in FIG. 6, the signal processing unit 133 generates material decomposition images using the plurality of images obtained from the correction unit 132. That is, the signal processing unit 133 generates the bone thickness image B and the soft-tissue thickness image S from the low-energy image L and the high-energy image H. There is a problem that these thickness images include more noise than the low-energy image L and the high-energy image H, which leads to degradation of image quality. In view of this, the signal processing unit 133 generates a filtered soft-tissue thickness image S' by applying filtering for reducing noise to the soft-tissue thickness image S. For the filtering, the signal processing unit 133 may use a Gaussian filter, a median filter, etc. Subsequently, in a manner similar to that in the description of FIG. 7, the signal processing unit 133 generates the accumulation image A from the low-energy image L and the high-energy image H. Furthermore, the signal processing unit 133 generates a bone thickness image B' with reduced noise from the filtered soft-tissue thickness image S' and the accumulation image A.

First, [Math. 10] below holds true, where $N_A(E)$ is the spectrum in the image that is the sum of the low-energy and high-energy X-rays, i.e., the accumulation image A, S is the soft-tissue thickness, and B is the bone thickness.

[Math. 10]

$$A = \frac{\int N_A(E)exp\{-\mu_B(E)B-\mu_S(E)S\}EdE}{\int N_A(E)EdE}$$

By substituting the soft-tissue thickness S and the pixel value A of the accumulation image at a given pixel into [Math. 10] and solving the non-linear equation, the bone thickness B at the given pixel can be derived. Here, by substituting the filtered soft-tissue thickness S' in place of the soft-tissue thickness S and solving [Math. 10], a bone thickness B' can be obtained.

Generally, since a soft-tissue image does not include so many highfrequency components, signal components are not readily lost even if filtering is applied to remove noise from a soft-tissue image. Thus, a bone thickness image B' with reduced noise can be obtained using the accumulation image A, which does not include much noise in the first place, and the soft-tissue thickness image S' with reduced noise. However, there is a problem that, in a case in which highfrequency components are included in the soft-tissue image, some signal components of the bone thickness image B' with reduced noise would be lost.

In such a case, the bone thickness image B' may be generated according to the block diagram of signal processing illustrated in FIG. 13. FIG. 13 illustrates a block diagram of signal processing according to the second embodiment. In the block diagram of signal processing illustrated in FIG. 13, in a similar manner as that in the signal processing in the block diagram illustrated in FIG. 12, the signal processing unit 133 generates material decomposition images using the plurality of images obtained from the correction unit 132. That is, the signal processing unit 133 generates the bone thickness image B and the soft-tissue thickness image S from the low-energy image L and the high-energy image H. In addition, the signal processing unit 133 generates the accumulation image A from the low-energy image L and the high-energy image H. Furthermore, the signal processing unit 133 generates an image that is the sum of the bone thickness image B and the soft-tissue thickness image S, i.e., the thickness image T. Then, the signal processing unit 133 generates a filtered thickness image T' by applying filtering for reducing noise to the thickness image T. Furthermore, the signal processing unit 133 generates a bone thickness image B' with reduced noise from the filtered thickness image T' and the accumulation image A.

Here, [Math. 11] below holds true when [Math. 10] is transformed based on T = B + S, where T is thickness.

[Math. 11]

$$A = \frac{\int N_A(E)exp\{-\mu_B(E)B-\mu_S(E)(T-B)\}EdE}{\int N_A(E)EdE}$$

By substituting the thickness T and the pixel value A of the accumulation image at a given pixel into [Math. 11] and solving the non-linear equation, the bone thickness B at the given pixel can be derived. Here, by substituting the filtered thickness T' in place of the thickness T and solving [Math. 11], a bone thickness B' can be obtained. As described in FIG. 9, the continuity in the thickness image T is high, and thus the thickness image T includes even less highfrequency components compared to the soft-tissue thickness image. Accordingly, signal components are not readily lost even if filtering is performed to remove noise. Thus, a bone thickness image B' with reduced noise can be obtained using the accumulation image A, which does not include much noise in the first place, and the thickness image T' with reduced noise.

Note that, while an example in which the bone thickness image B' with reduced noise is calculated is described in [Math. 11], the same applies to a case in which a soft-tissue thickness image with reduced noise is calculated. That is, the signal processing unit 133 can generate a material decomposition image of a first material (bone thickness image B') with reduced noise compared to a first material decomposition image (bone thickness image B) or a material decomposition image of a second material (soft-tissue thickness image S') with reduced noise compared to a second material decomposition image (soft-tissue thickness image S) based on a filtered thickness image T' and an accumulation image A obtained based on addition of a plurality of radiation images (H and L).

According to the present embodiment, material decomposition images with reduced noise can be obtained.

Furthermore, the results of the calculation in [Math. 11] can be stored in advance in a table in an internal memory of the signal processing unit 133, and the signal processing unit 133 can obtain the bone thickness image B' (soft-tissue thickness image S') corresponding to the filtered thickness image T' and the accumulation image A by referring to the table when performing the calculation in [Math. 11]. Thus, the signal processing unit 133 can obtain a material decomposition image (B' or S') with reduced noise for each material in a short amount of time in the capturing of moving images such as that in IVR, as well as in the capturing of still images.

Third Embodiment

In the third embodiment, a configuration for separating a contrast agent and reducing noise by utilizing the continuity of thickness illustrated in FIG. 9 will be described.

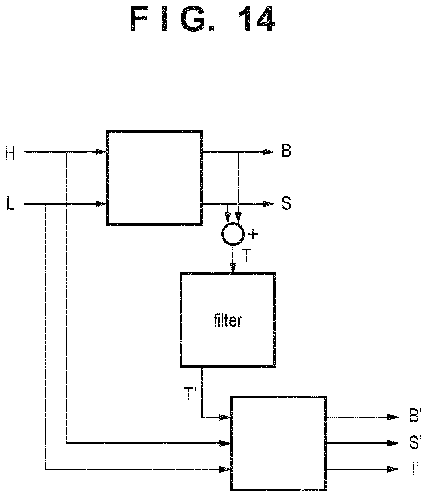

FIG. 14 illustrates a block diagram of signal processing according to the third embodiment. In the third embodiment, in a manner similar to that in the signal processing in FIG. 13, the signal processing unit 133 generates the bone thickness image B and the soft-tissue thickness image S from the low-energy image L and the high-energy image H. Furthermore, the signal processing unit 133 generates an image that is the sum of the bone thickness image B and the soft-tissue thickness image S, i.e., the thickness image T. Then, the signal processing unit 133 generates a filtered thickness image T' by performing filtering for removing the contrast-agent contrast on the thickness image T. Furthermore, the signal processing unit 133 generates a contrast-agent image I' from the filtered thickness image T', the low-energy image L, and the high-energy image H.

Here, [Math. 12] below holds true when [Math. 4] is expanded, where $\mu_I(E)$ is the linear attenuation coefficient of the contrast agent at energy E, and I is contrast-agent thickness.

[Math. 12]

$$L = \frac{\int N_L(E)exp\{-\mu_B(E)B-\mu_S(E)S-\mu_I(E)I\}EdE}{\int N_L(E)EdE}$$

$$H = \frac{\int N_H(E)exp\{-\mu_B(E)B-\mu_S(E)S-\mu_I(E)I\}EdE}{\int N_H(E)EdE}$$

Furthermore, [Math. 13] below holds true when [Math. 12] is transformed based on T = B + S + I, where T is the thickness in the thickness image.

[Math. 13]

$$L = \frac{\int N_L(E)exp\{-\mu_B(E)B-\mu_S(E)S-\mu_I(E)(T-B-S)\}EdE}{\int N_L(E)EdE}$$

$$H = \frac{\int N_H(E)exp\{-\mu_B(E)B-\mu_S(E)S-\mu_I(E)(T-B-S)\}EdE}{\int N_H(E)EdE}$$

By substituting the pixel value of the low-energy image L, the pixel value of the high-energy image H, and the thickness T in the thickness image at a given pixel into [Math. 13] and solving the non-linear system of equations, the thickness B in the bone thickness image and the thickness S in the soft tissue thickness image at the given pixel can be calculated. However, if calculation is directly performed in this state, the contrast-agent thickness I would always be 0 because the thickness image T is the sum of the bone thickness image B and the soft-tissue thickness image S, i.e., because T = B + S holds true.

As has been described in FIG. 10, bones disappear but contrast agents are visible in the thickness image T. That is, thickness changes only at blood-vessel portions including a contrast agent. However, in blood-vessel portions including a contrast agent, blood is replaced by the contrast agent. That is, regardless of whether or not a contrast agent is present, the true thickness image T' should not change.

Furthermore, since blood vessels including the contrast agent are generally thin, changes in thickness brought about by a contrast agent can be removed and the true thickness image T' can be obtained by performing filtering using a Gaussian filter or the like on the thickness image T. That is, by substituting the thickness in the filtered thickness image T' in place of that in the thickness image T into [Math. 13] and solving [Math. 13], the bone thickness image B', the soft tissue thickness image S', and the contrast-agent thickness image I' can be obtained.

The signal processing unit 133 generates a material decomposition image of a first material (bone thickness image B') with reduced noise compared to a first material decomposition image (bone thickness image B), a material decomposition image of a second material (soft tissue thickness image S') with reduced noise compared to a second material decomposition image (soft-tissue thickness image S), and a third material decomposition image (contrast-agent thickness image I') indicating a thickness of a third material (iodine-containing contrast agent) that differs from the first and second materials based on a filtered thickness image T' obtained by applying a spatial filter to a thickness image T, and a plurality of radiation images (low-energy image L and high-energy image H).

Here, the results of the calculation in [Math. 13] can be stored in advance in a table in the internal memory of the signal processing unit 133, and the signal processing unit 133 can obtain the bone thickness image B', the soft-tissue thickness image S', and the contrast-agent thickness image I' corresponding to the filtered thickness image T' and the plurality of radiation images (low-energy image L and high-energy image H) by referring to the table when performing the calculation in [Math. 13]. Thus, the signal processing unit 133 can acquire a material decomposition image (B', S', and I') for each material in a shorter amount of time compared to when a non-linear equation is analyzed.

In the signal processing in FIG. 14, noise is not reduced in the low-energy image L and the high-energy image H while noise is reduced in the filtered thickness image T'. Thus, there is a problem that the bone thickness image B', the soft tissue thickness image S', and the contrast-agent thickness image I' include much noise, which leads to degradation of image quality.

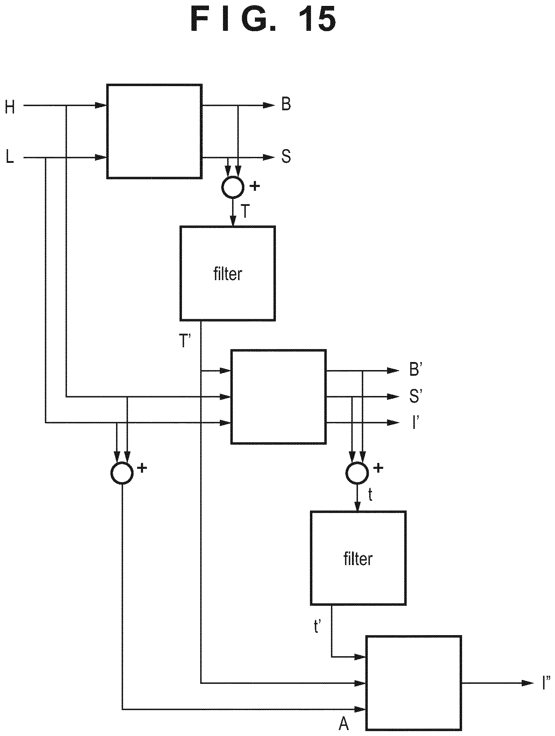

In this case, the signal processing unit 133 can also perform processing according to the block diagram of signal processing illustrated in FIG. 15. The signal processing unit 133 generates a filtered thickness image t' without the contrast agent by performing filtering for reducing noise on an image that is the sum of the bone thickness image B' and the soft tissue thickness image S', i.e., a thickness image t without the contrast agent. In addition, in a manner similar to that in the description of FIG. 7, the signal processing unit 133 generates the accumulation image A from the low-energy image L and the high-energy image H. Furthermore, the signal processing unit 133 generates a contrast-agent thickness image I" with reduced noise from the filtered thickness image T' (first thickness image), the filtered thickness image t' without the contrast agent (second thickness image), and the accumulation image A.

Here, [Math. 14] below holds true when [Math. 10] is expanded, where $\mu_I(E)$ is the linear attenuation coefficient of the contrast agent at energy E, and I is contrast-agent thickness.

[Math. 14]

$$A = \frac{\int N_A(E)exp\{-\mu_B(E)B - \mu_S(E)S - \mu_I(E)I\}EdE}{\int N_A(E)EdE}$$

Furthermore, [Math. 15] below holds true when [Math. 14] is transformed based on T = B + S + I and t = B + S, where T is the thickness and t is the thickness without the contrast agent.

[Math. 15]

$$A = \frac{\int N_A(E)exp\{-\mu_B(E)B - \mu_S(E)(t-B) - \mu_I(E)(T-t)\}EdE}{\int N_A(E)EdE}$$

By substituting the pixel value A of the accumulation image, the thickness in the thickness image T, and the thickness in the thickness image t without the contrast agent at a given pixel into [Math. 15] and solving the non-linear equation, the thickness in the bone thickness image B at the given pixel can be calculated. However, if calculation is directly performed in this state, the contrast-agent thickness I would always be 0 because the thickness image T is the sum of the bone thickness image B and the soft-tissue thickness image S, i.e., because T = B+S holds true.

However, by substituting the filtered thickness T' in place of the thickness image T and the filtered thickness image t' without the contrast agent in place of the thickness image t without the contrast agent into [Math. 15], a contrast-agent thickness image I" with reduced noise can be calculated. As described in FIG. 9, the continuity of the thickness image T is high, and thus signal components are not readily lost from the thickness image T even if filtering is performed to reduce noise. The same applies to the thickness image t without the contrast agent. In such a manner, the contrast-agent thickness image I" with reduced noise can be obtained using the accumulation image A, which does not include much noise in the first place, the thickness image t' without the contrast agent and with reduced noise, and the thickness image T' with reduced noise.

That is, the signal processing unit 133 generates a second thickness image (thickness image t without the contrast agent) without a third material (iodine-containing contrast agent) based on a material decomposition image of a first material with reduced noise (bone thickness image B') and a material decomposition image of a second material with reduced noise (soft-tissue thickness image S'). Furthermore, the signal processing unit 133 generates a material decomposition image of a third material (contrast-agent thickness image I") with reduced noise compared to a third material decomposition image (contrast-agent thickness image I') based on a filtered first thickness image T' obtained by applying a spatial filter to a thickness image T, a filtered second thickness image t' obtained by applying a spatial filter to the second thickness image t, and an accumulation image A obtained based on addition of a plurality of radiation images.

Here, the results of the calculation in [Math. 15] can be stored in advance in a table in the internal memory of the signal processing unit 133, and the signal processing unit 133 can obtain the contrast-agent thickness image I" with reduced noise corresponding to the filtered first thickness image T', the accumulation image A, and the filtered second thickness image t' by referring to the table when performing the calculation in [Math. 15]. Thus, the signal processing unit 133 can acquire a material decomposition image of the contrast agent with reduced noise in a shorter amount of time compared to when a non-linear equation is analyzed.

Note that, in the first to third embodiments above, an indirect-type X-ray sensor using a fluorescent material is used as the X-ray imaging apparatus 104. However, the present invention is not limited to such an embodiment. For example, a direct-type X-ray sensor using a direct conversion material such as CdTe may be used. That is, the X-ray sensor may be that of the indirect type or the direct type.

Furthermore, in the first to third embodiments, the tube voltage of the X-ray generation apparatus 101 is changed in the operation in FIG. 4, for example. However, the present invention is not limited to such an embodiment. The energy of the X-rays to which the X-ray imaging apparatus 104 is exposed may be changed by temporally switching filters of the X-ray generation apparatus 101. That is, there is no limitation whatsoever regarding the method for changing the energy of the X-rays to which the X-ray imaging apparatus 104 is exposed.

Furthermore, while images with different energies were obtained by changing the X-ray energy in the first to third embodiments, the present invention is not limited to such an embodiment. For example, a stacked configuration may be adopted in which a plurality of fluorescent materials 105 and a plurality of two-dimensional detectors 106 are stacked, whereby images with different energies are respectively obtained from the two-dimensional detectors on the front and back sides relative to the X-ray incidence direction.

Furthermore, in the first to third embodiments, energy subtraction processing is performed using the imaging control apparatus 103 of the X-ray image-capturing system. However, the present invention is not limited to such an embodiment. For example, images obtained by the imaging control apparatus 103 may be transferred to a different computer, where energy subtraction processing is performed. For example, a configuration may be adopted in which obtained images are transferred to a different personal computer via a medical PACS to be displayed after energy subtraction processing is performed. That is, the apparatus in which the correction processing described in the embodiments is performed need not be paired with an image-capturing apparatus (i.e., may be an image viewer).

According to the present embodiment, material decomposition images with reduced noise can be obtained. Furthermore, contrast agents and medical devices can also be separated while estimating bone thickness and soft-tissue thickness with reduced noise.

Other Embodiments

The present invention can be implemented by processing of supplying a program for implementing one or more functions of the above-described embodiments to a system or apparatus via a network or storage medium, and causing one or more processors in the computer of the system or apparatus to read out and execute the program. The present invention can also be implemented by a circuit (for example, an ASIC) for implementing one or more functions.

This application claims priority from Japanese Patent Application No. 2019-159726 filed on September 2, 2019.

**Claims**

1. An image processing apparatus (103), comprising

generating means (131, 133) for generating, using a plurality of radiation images corresponding to mutually different radiation energies, a first material decomposition image (B) that indicates a thickness of a first material and a second material decomposition image (S) that indicates a thickness of a second material that differs from the first material,
wherein the generating means generates, using the first material decomposition image and the second material decomposition image, a thickness image (T) in which the thickness of the first material and the thickness of the second material are added together;
**characterized in that**
the generating means, using a new thickness image (T') obtained by applying a spatial filter to the thickness image, and the plurality of radiation images,
generates a material decomposition image (B') indicating a thickness of the first material with reduced noise compared to the first material decomposition image, a material decomposition image (S') indicating a thickness of the second material with reduced noise compared to the second material decomposition image, and a third material decomposition image (I') that indicates a thickness of a third material that is different from the first and second materials.

2. The image processing apparatus according to claim 1, **characterized in that** the first material includes at least one of calcium, hydroxyapatite, or bone, and the second material includes at least one of water or fat.

3. The image processing apparatus according to claim 1 or 2, **characterized by** further comprising

obtaining means for obtaining the plurality of radiation images by capturing images at a first energy and at a second energy that is higher than the first energy,
wherein the average energy of a spectrum of radiation for obtaining a radiation image based on the first energy is an energy lower than the iodine K-edge (1130).

4. The image processing apparatus according to claim 3, **characterized in that** the obtaining means obtains, as the plurality of radiation images, radiation images that are obtained by performing sampling and holding multiple times during exposure to one shot of radiation.

5. The image processing apparatus according to any one of claims 1 to 4, **characterized in that** the generating means generates, using the new thickness image obtained by applying the spatial filter to the thickness image, and the plurality of radiation images, the third material decomposition image that indicates a thickness of the third material that is different from the first and second materials and a further thickness image (t) without the third material based on the material decomposition image of the first material with reduced noise and the material decomposition image of the second material with reduced noise.

6. The image processing apparatus according to claim 5, **characterized in that**:

the generating means, based on the new thickness image (T') obtained by applying a spatial filter to the thickness image (T),
a filtered further thickness image (t') obtained by applying a spatial filter to the further thickness image (t), and
an accumulation image (A) obtained based on addition of the plurality of radiation images,
generates a material decomposition image (I") of the third material with reduced noise compared to the third material decomposition image (I).

7. The image processing apparatus according to claim 5 or 6, **characterized in that** the third material includes an iodine-containing contrast agent.

8. The image processing apparatus according to any one of claims 1 to 4, **characterized in that** the generating means generates, using the thickness image and an accumulation image obtained by addition of the plurality of radiation images,
the material decomposition image indicating a thickness of the first material with reduced noise compared to the first material decomposition image, and the material decomposition image indicating a thickness of the second material with reduced noise compared to the second material decomposition image.

9. A radiation imaging system **characterized by** comprising:

the image processing apparatus according to any one of claims 1 to 8; and
a radiation imaging apparatus (101, 104) that generates the plurality of radiation images by exposure to radiation.

10. An image processing method for processing radiation images, comprising

generating, using a plurality of radiation images corresponding to mutually different radiation energies, a first material decomposition image that indicates a thickness of a first material and a second material decomposition image that indicates a thickness of a second material that differs from the first material; and
generating, using the first material decomposition image and the second material decomposition image, a thickness image in which the thickness of the first material and the thickness of the second material are added together;
**characterized in that**
the generating, using a new thickness image obtained by applying a spatial filter to the thickness image, and the plurality of radiation images,
generates a material decomposition image indicating a thickness of the first material with reduced noise compared to the first material decomposition image, a material decomposition image indicating a thickness of the second material with reduced noise compared to the second material decomposition image, and a third material decomposition image that indicates a thickness of a third material that is different from the first and second materials.

11. A program for causing a computer to execute the method according to claim 10.

**Patentansprüche**

1. Bildverarbeitungsvorrichtung (103), umfassend

eine Erzeugungseinrichtung (131, 133) zum Erzeugen, unter Verwendung mehrerer Strahlungsbilder, die voneinander verschiedenen Strahlungsenergien entsprechen, eines ersten Materialzerlegungsbilds (B), das eine Dicke eines ersten Materials angibt, und eines zweiten Materialzerlegungsbilds (S), das eine Dicke eines vom ersten Material verschiedenen zweiten Materials angibt,
wobei die Erzeugungseinrichtung unter Verwendung des ersten Materialzerlegungsbilds und des zweiten Materialzerlegungsbilds ein Dickenbild (T) erzeugt, in dem die Dicke des ersten Materials und die Dicke des zweiten Materials addiert sind;
**dadurch gekennzeichnet, dass**
die Erzeugungseinrichtung unter Verwendung eines durch Anwenden eines räumlichen Filters auf das Dickenbild erhaltenen neuen Dickenbilds (T') und der mehreren Strahlungsbilder
erzeugt: ein Materialzerlegungsbild (B'), das eine Dicke des ersten Materials mit im Vergleich zum ersten Materialzerlegungsbild verringertem Rauschen angibt, ein Materialzerlegungsbild (S'), das eine Dicke des zweiten Materials mit im Vergleich zum zweiten Materialzerlegungsbild verringertem Rauschen angibt, und ein drittes Materialzerlegungsbild (I'), das eine Dicke eines vom ersten und dem zweiten Material verschiedenen dritten Materials angibt.

2. Bildverarbeitungsvorrichtung nach Anspruch 1, **dadurch gekennzeichnet, dass**
das erste Material Calcium, Hydroxyapatit und/oder Knochen enthält und das zweite Material Wasser und/oder Fett enthält.

3. Bildverarbeitungsvorrichtung nach Anspruch 1 oder 2, **dadurch gekennzeichnet, dass** sie ferner umfasst

eine Erfassungseinrichtung zum Erfassen der mehreren Strahlungsbilder durch Aufnehmen von Bildern mit einer ersten Energie und mit einer zweiten Energie, die höher als die erste Energie ist, wobei die durchschnittliche Energie eines Strahlungsspektrums zum Erfassen eines Strahlungsbilds basierend auf der ersten Energie eine Energie ist, die niedriger als die K-Absorptionskante von Iod (1130) ist.

4. Bildverarbeitungsvorrichtung nach Anspruch 3, **dadurch gekennzeichnet, dass** die Erfassungseinrichtung als die mehreren Strahlungsbilder Strahlungsbilder erfasst, die durch mehrmaliges Durchführen von Abtasten und Halten während der Bestrahlung mit einer Strahlungseinheit erfasst werden.

5. Bildverarbeitungsvorrichtung nach einem der Ansprüche 1 bis 4, **dadurch gekennzeichnet, dass** die Erzeugungseinrichtung unter Verwendung des durch Anwenden des räumlichen Filters auf das Dickenbild erhaltenen neuen Dickenbilds und der mehreren Strahlungsbilder erzeugt: das dritte Materialzerlegungsbild, das eine Dicke des vom ersten und dem zweiten Material verschiedenen dritten Materials angibt, und ein weiteres Dickenbild (t) ohne das dritte Material basierend auf dem Materialzerlegungsbild des ersten Materials mit reduziertem Rauschen und dem Materialzerlegungsbild des zweiten Materials mit reduziertem Rauschen.

6. Bildverarbeitungsvorrichtung nach Anspruch 5, **dadurch gekennzeichnet, dass**:

   die Erzeugungseinrichtung, basierend auf dem durch Anwenden eines räumlichen Filters auf das Dickenbild (T) erhaltenen neuen Dickenbild (T'),
   einem durch Anwenden eines räumlichen Filters auf das weitere Dickenbild (t) erhaltenen gefilterten weiteren Dickenbild (t'), und
   einem basierend auf einer Addition der mehreren Strahlungsbilder erhaltenen Akkumulationsbild (A),
   ein Materialzerlegungsbild (I") des dritten Materials mit im Vergleich zum dritten Materialzerlegungsbild (I) verringertem Rauschen erzeugt.

7. Bildverarbeitungsvorrichtung nach Anspruch 5 oder 6, **dadurch gekennzeichnet, dass** das dritte Material ein iodhaltiges Kontrastmittel enthält.

8. Bildverarbeitungsvorrichtung nach einem der Ansprüche 1 bis 4, **dadurch gekennzeichnet, dass** die Erzeugungseinrichtung unter Verwendung des Dickenbilds und eines durch Addition der mehreren Strahlungsbilder erhaltenen Akkumulationsbilds erzeugt:
   das Materialzerlegungsbild, das eine Dicke des ersten Materials mit im Vergleich zum ersten Materialzerlegungsbild verringertem Rauschen angibt, und das Materialzerlegungsbild, das eine Dicke des zweiten Materials mit im Vergleich zum zweiten Materialzerlegungsbild verringertem Rauschen angibt.

9. Strahlungsbildgebungssystem, **dadurch gekennzeichnet, dass** es umfasst:

   die Bildverarbeitungsvorrichtung nach einem der Ansprüche 1 bis 8; und
   eine Strahlungsbildgebungsvorrichtung (101, 104), die die mehreren Strahlungsbilder durch Bestrahlung mit Strahlung erzeugt.

10. Bildverarbeitungsverfahren zum Verarbeiten von Strahlungsbildern, umfassend

   Erzeugen, unter Verwendung mehrerer Strahlungsbilder, die voneinander verschiedenen Strahlungsenergien entsprechen, eines ersten Materialzerlegungsbilds, das eine Dicke eines ersten Materials angibt, und eines zweiten Materialzerlegungsbilds, das eine Dicke eines vom ersten Material verschiedenen zweiten Materials angibt; und
   Erzeugen, unter Verwendung des ersten Materialzerlegungsbilds und des zweiten Materialzerlegungsbilds, eines Dickenbilds, in dem die Dicke des ersten Materials und die Dicke des zweiten Materials addiert sind;
   **dadurch gekennzeichnet, dass**
   das Erzeugen, unter Verwendung eines durch Anwenden eines räumlichen Filters auf das Dickenbild erhaltenen neuen Dickenbilds und der mehreren Strahlungsbilder,
   erzeugt: ein Materialzerlegungsbild, das eine Dicke des ersten Materials mit im Vergleich zum ersten Materialzerlegungsbild verringertem Rauschen angibt, ein Materialzerlegungsbild, das eine Dicke des zweiten Materials mit im Vergleich zum zweiten Materialzerlegungsbild verringertem Rauschen angibt, und ein drittes Materialzerlegungsbild, das eine Dicke eines vom ersten und zweiten Material verschiedenen dritten Materials angibt.

**11.** Programm zum Veranlassen eines Computers, das Verfahren nach Anspruch 10 auszuführen.

**Revendications**

**1.** Appareil de traitement d'image (103) comprenant

un moyen de génération (131, 133) destiné à générer, en utilisant une pluralité d'images de rayonnement correspondant à des énergies de rayonnement mutuellement différentes, une première image de décomposition de matériau (B) qui indique une épaisseur d'un premier matériau et une deuxième image de décomposition de matériau (S) qui indique une épaisseur d'un deuxième matériau qui diffère du premier matériau, dans lequel le moyen de génération génère, en utilisant la première image de décomposition de matériau et la deuxième image de décomposition de matériau, une image d'épaisseur (T) dans laquelle l'épaisseur du premier matériau et l'épaisseur du deuxième matériau sont ajoutées l'une à l'autre ; **caractérisé en ce que** le moyen de génération, en utilisant une nouvelle image d'épaisseur (T') obtenue par application d'un filtre spatial à l'image d'épaisseur, et la pluralité d'images de rayonnement, génère une image de décomposition de matériau (B') indiquant une épaisseur du premier matériau avec un bruit réduit par comparaison à la première image de décomposition de matériau, une image de décomposition de matériau (S') indiquant une épaisseur du deuxième matériau avec un bruit réduit par comparaison à la deuxième image de décomposition de matériau, et une troisième image de décomposition de matériau (I') qui indique une épaisseur d'un troisième matériau qui est différent des premier et deuxième matériaux.

**2.** Appareil de traitement d'image selon la revendication 1, **caractérisé en ce que** le premier matériau comprend au moins l'un du calcium, de l'hydroxyapatite ou de l'os, et le deuxième matériau comprend au moins l'une de l'eau ou d'une graisse.

**3.** Appareil de traitement d'image selon la revendication 1 ou 2, **caractérisé en ce qu'**il comprend en outre

un moyen d'obtention destiné à obtenir la pluralité d'images de rayonnement en capturant des images à une première énergie et à une deuxième énergie plus élevée que la première énergie, dans lequel l'énergie moyenne d'un spectre de rayonnement permettant d'obtenir une image de rayonnement sur la base de la première énergie est une énergie inférieure à la raie K de l'iode (1130).

**4.** Appareil de traitement d'image selon la revendication 3, **caractérisé en ce que** le moyen d'obtention obtient, en tant que pluralité d'images de rayonnement, des images de rayonnement qui sont obtenues en effectuant un échantillonnage et un maintien de multiples fois pendant l'exposition à un tir de rayonnement.

**5.** Appareil de traitement d'image selon l'une quelconque des revendications 1 à 4, **caractérisé en ce que** le moyen de génération génère, en utilisant la nouvelle image d'épaisseur obtenue par application du filtre spatial à l'image d'épaisseur, et la pluralité d'images de rayonnement, la troisième image de décomposition de matériau qui indique une épaisseur du troisième matériau qui est différente des premier et deuxième matériaux et une autre image d'épaisseur (t) sans le troisième matériau sur la base de la première image de décomposition de matériau avec un bruit réduit et la deuxième image de décomposition de matériau avec un bruit réduit.

**6.** Appareil de traitement d'image selon la revendication 5, **caractérisé en ce que** :

le moyen de génération, sur la base de la nouvelle image d'épaisseur (T') obtenue par application d'un filtre spatial à l'image d'épaisseur (T), d'une autre image d'épaisseur filtrée (t') obtenue par application d'un filtre spatial à l'autre image d'épaisseur (t), et d'une image d'accumulation (A) obtenue sur la base d'une addition de la pluralité d'images de rayonnement, génère une image de décomposition de matériau (I') du troisième matériau avec un bruit réduit par comparaison à la troisième image de décomposition de matériau (I).

**7.** Appareil de traitement d'image selon la revendication 5 ou 6, **caractérisé en ce que** le troisième matériau comprend un agent de contraste contenant de l'iode.

**8.** Appareil de traitement d'image selon l'une quelconque des revendications 1 à 4, **caractérisé en ce que** le moyen de génération génère, en utilisant l'image d'épaisseur et une image d'accumulation obtenue par addition de la pluralité d'images de rayonnement,

l'image de décomposition de matériau indiquant une épaisseur du premier matériau avec un bruit réduit par comparaison à la première image de décomposition de matériau, et l'image de décomposition de matériau indiquant une épaisseur du deuxième matériau avec un bruit réduit par comparaison à la deuxième image de décomposition de matériau.

9. Système d'imagerie de rayonnement **caractérisé en ce qu'**il comprend :

l'appareil de traitement d'image selon l'une quelconque des revendications 1 à 8 ; et
un appareil d'imagerie de rayonnement (101, 104) qui génère la pluralité d'images de rayonnement par exposition à un rayonnement.

10. Procédé de traitement d'image destiné à traiter des images de rayonnement, comprenant

la génération, en utilisant une pluralité d'images de rayonnement correspondant à des énergies de rayonnement mutuellement différentes, d'une première image de décomposition de matériau qui indique une épaisseur d'un premier matériau et d'une deuxième image de décomposition de matériau qui indique une épaisseur d'un deuxième matériau qui diffère du premier matériau ; et

la génération, en utilisant la première image de décomposition de matériau et la deuxième image de décomposition de matériau, une image d'épaisseur dans laquelle l'épaisseur du premier matériau et l'épaisseur du deuxième matériau sont ajoutées l'une à l'autre ;

**caractérisé en ce que**

la génération, en utilisant une nouvelle image d'épaisseur obtenue par application d'un filtre spatial à l'image d'épaisseur, et la pluralité d'images de rayonnement,

génère une image de décomposition de matériau indiquant une épaisseur du premier matériau avec un bruit réduit par comparaison à la première image de décomposition de matériau, une image de décomposition de matériau indiquant une épaisseur du deuxième matériau avec un bruit réduit par comparaison à la deuxième image de décomposition de matériau, et une troisième image de décomposition de matériau qui indique une épaisseur d'un troisième matériau différent des premier et deuxième matériaux.

11. Programme destiné à amener un ordinateur à mettre en oeuvre le procédé selon la revendication 10.

# F I G. 1

F I G. 2

# FIG. 3

X-RAY  301 302 303

SYNCHRO-NIZATION SIGNAL  307

Reset

SH_N  $R_1$  0

SH_S  $R_1+B$  $R_1+B+R_2$

READ

S–N
$R_1+B-R_1$  304
B

S–N
$R_1+B+R_2-0$  306
$R_1+B+R_2$

305

$R_1+B+R_2-B = R_1+R_2$

TIME

EP 4 014 874 B1

# FIG. 4

EP 4 014 874 B1

# F I G. 5

# F I G. 6

# F I G. 7

# FIG. 8

CONTRAST AGENT  SOFT TISSUE

CONTRAST AGENT

BONE  STENT

BONE  STENT

ACCUMULATION IMAGE A

BONE IMAGE B

EP 4 014 874 B1

# F I G. 9

SOFT-TISSUE IMAGE S

THICKNESS IMAGE T

EP 4 014 874 B1

# F I G. 10

ACCUMULATION IMAGE A

THICKNESS IMAGE T

EP 4 014 874 B1

# FIG. 11

**11A**

X-RAY SPECTRA

SPECTRA [%]

ENERGY [keV]

---- 50kV
—— 120kV

1110  1120
1101
1102

**11B**

MATERIAL LINEAR ATTENUATION COEFFICIENT

LINEAR ATTENUATION
COEFFICIENT
[cm⁻¹]

ENERGY [keV]

—— SOFT TISSUE
---- BONE
—— CONTRAST
AGENT

1130
1105
1104
1103

EP 4 014 874 B1

# F I G. 12

# F I G. 13

# F I G.  14

# F I G. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H3285475 A
- US 20130129180 A1
- US 20110019891 A1
- JP 2019159726 A